# EUROPEAN PATENT SPECIFICATION

(11) **EP 2 505 634 B1**
(45) Date of publication and mention of the grant of the patent: **04.12.2013**
(21) Application number: 11425082.2
(22) Date of filing: 31.03.2011
(51) Int. Cl.: C12M 1/00, C12M 1/02, B01F 11/00

(54) **A solid state fermentation plant**
Festphasen-Fermentationsvorrichtung
Installation pour la fermentation en milieu solide

(43) Date of publication of application: 03.10.2012
(73) Proprietor: Technelep srl, 20152 Milano (IT)
(72) Inventor: Bobbiesi, Giuseppe, 20152 Milano (IT); Bobbiesi, Marco, 20152 Milano (IT)
(74) Representative: Beneduce, Gianna

(56) References cited:
- WO-A1-2004/111181
- US-A- 3 753 582
- US-A1- 2002 009 803
- US-B1- 6 190 913
- US-B1- 6 245 555
- US-B1- 6 620 614
- US-B1- 6 644 341

## Description

### Short description of the invention.

It is the object of the present invention a solid state fermentation plant for the production, in sterility conditions and high concentration, of vital microorganism cultures, such as, for example, eumycetes, basidiumycetes, bacteria, actinomycetes, bacilli, vital spores of the above mentioned microorganisms, enzymes, secondary metabolites, Said plant, in comparison with other similar plants and known devices, is characterized by a remarkable structural simplicity and reliability.

### State of the art

The solid state fermentation, internationally SSF initialled, is followed with more and more interest because of the numerous advantages it presents in comparison with the submerged fermentation, internationally initialled SmF. Said advantages are identifiable in: higher productivity, higher final concentration of the desired products, higher production stability, lower catabolic repression, capability of using also water insoluble substrata, lower sterility requirements thanks to the lower presence of available water, very low downstream to obtain the finished products, practically no effluents to be treated, lower drying costs for the products to be dried.

Numerous documents exist relevant to the solid state fermentation; among them, for structural affinity, the plants described in the following patent publications are considered. WO-A 2004111B1 describes a solid state fermentation plant wherein the fermentation is carried out in plastic material bags, each one of them being subdivided in two superimposed, one to the other, chambers, by means of a partially perforated diaphragm, horizontally located in the median position of the bag, through said diaphragm the air coming from a filter flows, one filter for each bag. Both inoculum and air are introduced into the bag containing the culture medium through some nozzles located on the external bag surface. In comparison with the fermentation plant of the above mentioned patent application, the plant bag of the present invention is simpler, not having any subdivision element, but it guarantees, due to the nozzle lack, a higher facility in obtaining the full plant sterility and therefore lower operation costs.

Another document of the known art is represented by the US patent n°6,620,614 wherein a fermenter is described essentially formed by an equipment inside of which a certain number of plates is present, superimposed one to the other, carrying the fermentation medium to be inoculate. Inoculum is introduced into the medium starting from the equipment upper plate. The fermenter closed structure doesn't allow, from outside, the visual control of the fermentation proceeding and the pollution, if any, even of only a single plate, implies the pollution also of all the other plates. Compared to the above described fermenter, the fermentation bag of the present invention has a simpler structure; the possibility of a visual control exists and therefore, when necessary, to intervene, so assuring the maximum sterility.

Finally, US Patent n°6,190,913, describes a fermentation apparatus in liquid phase placed on a swinging platform. Said fermenter, if applied as such in the solid state fermentation, would not give positive results because, for some preparation, felts or panels would be obtained, that would crumble without flowing any longer. Furthermore, the compression system by means of belts has the only purpose of maintaining in position the fermentation bags and not to help in maintaining a certain air velocity in the upper part of the horizontally placed bag, as in the present patent application.

US 2002/009803 A 1 describes a method and an apparatus for cultivation of sensitive cells and/or tissues such as bacteria, yeasts and fungi in a container comprising a culture medium, a gas atmosphere and the cells or tissues to be cultured, said container being immersed or submerged in a thermostatically controlled liquid bath to such an extent that only the uppermost top-part of the container is above the upper surface of the liquid.

The apparatus of US 2002/009803 A1 comprises a tank to be filled with a liquid, means to heat said liquid, means for controlling the temperature of the liquid and means for stirring said liquid: it has a roof with openings through one of which the container is immersed or submerged.

US 6,245,555 describes a method and an apparatus for culturing cells, tissues and/or microorganisms including bacteria and fungi. The process is developed in a reservoir or a bioreactor consisting in an open liner with a V-shaped bottom contained in a cylindrical vessel: a biomass dispersion is introduced in the liner and the inocolum as well. Sterilized air is introduced in the lowest point of the reservoir in order to aerate the biomass.

The apparatus of US 6,245,555 consists in a disposable liner contained in a vessel whose cover closes contemporarily both the vessel and the liner.

The introduction of the inocolum and air takes place through nozzles located on the cover. A third nozzle allows to locate a sampling tube.

After use, the liner is disposed while the wessel can be reused with a new liner.

### Detailed description of the invention

The object of the present invention is formed by a multifunctional plant of solid state fermentation, that requires a low investment cost for the production of various genera and species of vital microorganisms, vital spores, vital mycelia, enzymes, spices, vitamins, antibiotics and other secondary metabolites. Said multifunctional solid state fermentation plant is characterized by a high productivity, great operation simplicity, absolute sterility, full temperature control, and by an industrial automatically dosed inoculum.

Particularly, the solid state fermentation plant, object of the present invention, is formed by a scaffolding (2) bearing some crossbars on which the shelves (1) rest, in turn bearing the bags (7) made of autoclave, transparent, plastic material, in a number proportional to the shelves (1), in which the substratum or fermentation medium (12) is contained, said scaffolding (2) being tamponed on the perimetric walls and on the ceiling to form a closed chamber with suitable access doors; by the shelves (1), in variable number, depending on the plant size, that are provided with an interspace or a coil wherein a thermostabilizing fluid is made to flow, so to thermostabilize, contemporarily, the bag (7), supported on the shelf (1), and the closed chamber containing the scaffolding (2) where the fermentation takes place; by a suitable tank for the preparation and storage of the above mentioned thermostabilizing fluid, said tank being provided with a pump for the delivery of said fluid to the shelves (1); by control and regulation instruments and by suitable distribution and return manifolds of said fluid; by the steam autoclave (24) for the sterilization ad cooking, if any, when substrata are used that need to be cooked, provided with a rack or scaffold (25) carrying trays (23), capable of bearing as many trays (23) as the bags (7) are that are to be sterilized or cooked for every production cycle; by the automatic device or gun (26) for the inoculum injection, in an automatically predetermined quantity, into the hermetically closed bag (7), already containing the culture medium (12), previously sterilized in the autoclave (24); by a production and delivery system to the plant of humid (14) and/or dry (13) air, under slight pressure, to be insufflate, alternatively, into the bags (7) through a series of filter (15); said series of filter (15) for sterilized air being formed by a metallic container with a replaceable sterilizing cartridge (16), of 2" in diameter and 5" in height, of suitable material, proportional to the number of plant bags (7), said filters (15) being provided with manifolds (17) of the sterilized air exit, that carry as many connections as the pipes of flexible and steam sterizable in autoclave material contemporary to the filters (15), each pipe ending with the valve (10) and the metallic nozzle (9), also flame sterilzable, for the injection of sterilized air in the already sterilized and inoculated bag (7); by the device (6) for maintaining a reduced cross section of the bag during fermentation or drying when, into the same, fermentation or drying air is insufflate, that tends to inflate it; by the discharge system (8) for every bag (7) of the fermentation or drying insufflated air, easily fixable on the same and able to avoid the pollutants entry into the same during fermentation and/or drying and to promote the discharge of the carbon dioxide that forms during the fermentation.

The scaffolding (2), bearing the shelves (1), can be made in different materials, preferably of metallic prefabricated type, with the shelves (1) at a suitable distance in height, preferably 40 cm, with easy access to all the shelves and levels by means of balconies, if any, at requested height. Said scaffolding (2) is closed, at its sides, preferably with transparent and termically insulating material like, for example, alveolate polycarbonate, and is provided with the necessary entry doors, suitable to introduce or remove the trays (23) and the bags (7), so as to serve as constant-temperature chamber. The scaffolding (2) is suitable to contain the thermostabilizing liquid pipes, the temperature control batteries of dry and humid air, the sterilizing filters (15) with the relevant distribution pipes of the sterilized air to the bags (7), during fermentation and/or drying, placed on the shelves (1) of the scaffolding (2).

The shelves (1) are metallic, preferably in stainless steel, and have in their lower part a countershelf fixed so as to form a watertight interspace, with internally a number of buffles (5) suitable to improve the flowing velocity of the thermostabilizing fluid. The shelves (1) are provided with a lower pipe (3) bringing the thermostabilizing fluid to one interspace extremity and with a pipe (4) located on the upper surface in a diagonally opposed position to the lower pipe to allow the thermostabilizing fluid exit. The two pipes (3) and (4) are connected to the manifold bringing the thermostabilizing fluid and to the manifold collecting and directing it to the starting tank, respectively. Alternatively to the interspace, a coil located under every shelf (1) may be used.

The two bringing and return manifolds of the thermostabilizing fluid allow a uniform fluid flow to all the shelves (1) and the recycle to the tank of the thermostabilizing fluid, wherein the filling up, if any, or the overflow discharge and, especially, the fluid temperature control, by suitable instrumentation, are assured. The recycle is assured by a suitable motor pump that sucks from the tank, not indicated in the attached Figures, and delivers to the bringing tube manyfolds the termostabilizing fluid by distributing it to the shelves (1) in an optimal manner.

The fermentation plant of the present invention is provided with the autoclave (24) for cooking and/or steam sterilisation, formed by a parallelepipedal, horizontal chamber, which contains a rack or a scaffold (25), bearing trays (23) these last ones being in metallic sheet, preferably perforated and in stainless steel, of such sizes to contain the fermentation bags, already filled with the culture medium and hermetically closed, preferably by electrowelding. The autoclave (24) is provided with all the instrumentation and automatisms necessary to perform cooking and/or sterilization cycles of the substratum (12), contained in the bags (7) in a uniform manner and in short times, with the trays (23) containing the bags (7) separated one from the other, and with a large contact surface exposed to the steam compared to the quantity of material contained in the bag. The introduction of the bags into the autoclave and their drawing out takes place by means of the trays, so avoiding possible handling difficulties and/or bag damages, if any. The inoculum of the bags (7) containing the culture medium (12), is performed with a special gun (26), on purpose manufactured, in stainless steel, having a double barrel (30), the external barrel having the purpose of protecting from heat the internal one during the flame sterilization, the internal barrel ending with the nozzle (29) having the lock (28) connected to a lever (38) handling system with return spring (35); the gun (26) being also provided with the connections for two tubes (31) and (32), bringing the inoculum and the sterile pressurized air, respectively, and a rear seal system formed by an oil or other high boiling liquid tank with two O-rings (27) on the lock 28. The gun (26) is fixed on the upper part to a rail (33) on which it can run backwards and forwards, horizontally, and even with a certain slope, carried by a truck (34) sliding on the same rail (33). The displacements can be driven by the rudder bar (40) connected to the steel cable (37), coated by a flexible metallic sheath and by a return spring (36). A microswitch, suitably placed, allows to send an electric signal to a device, if any, for delivering inoculum and sterile air following the gun sliding. A stop (39), suitably placed, implements the lock (28) automatic stop at a desired point of the gun run on the rail (33), allowing the inoculum spray, together with sterile air, on the culture medium (12).

The equipment for the production of humid and dry air for the insufflation into the bags (7) is formed by: a filter (19) with large surface with filtration degree of 1 micron, with a further external filtering and protecting panel, made in rock wool, a cooling battery (18) able to work with a non freezing cooling fluid, an automatic chiller group to supply the cooling fluid into the battery (18), an absorption automatically regenerating dehumidifier (20) using a portion of the entering air, an automatically controlled humidifier to assure the desired degree of humidity of the humid air, a blower (22) with lateral channels, low humid air suction and delivery pressure, a blower (21) lateral channels, low dry air suction and delivery pressure, piping, a final battery of humid air thermostabilizing, a final battery of dry air thermostabilizing, an air humidity controller, temperature controllers, manometers, automatic over pressure and over flow rate valves. The connecting pipes and valves of the above different components are manufactured in such a way to allow optimal flowrates, to avoid excessive negative or over pressures, to maximize the drying degree of the dry air, to recover the rigeneration air of the dehydrator, already humid, and to automatically correct its humidity degree, so as to always obtain the optimal prefixed humidity degree.

The filters (15) are fixed on the structure of the fermentation plant in a suitable position to easily serve, each one, several bags, preferably eight bags for filter.

The bags (7) are preferably in polyamide and of large capacity, but still manageable by a single operator; they can be filled through the mouth and hermetically closed by thermowelding. Their preferable sizes are 115 x 45 cm, to be loaded with 10 - 20 kg of culture medium and, preferable 15 kg with a culture medium having specific weight between 2 and 3 kg/dm³.

The culture medium (12) is disposed inside the bag (7), on the whole surface of the lying bag, in uniform and thin layer ready for the sterilization, the inoculum and the subsequent fermentation and/or drying and the bag, so prepared, is placed, well laid, on a tray (23) of suitable size.

The bags (7) so prepared and laid on the respective trays (23), are placed in the autoclave (24) and sterilized. At the end of the sterilization the autoclave (24) is opened and the bags are left to cool down to 30-35°C; then the trays containing the bags are drawn out from the autoclave and placed on a plane for the inoculum.

Inoculation is performed by means of the gun (26) that assures a widespread distribution of the liquid inoculum coming, in sterile conditions, from the fermenter for submerged fermentation or a suitable pressurized tank, and can also provide for the dosage of the inoculum quantity by means of the signal sent by the microswitch on the gun, with the following procedure: the double barrel (30) of the gun (26) is heated by the flame to be sterilized till reaching a sufficient temperature to melt the bag material. The gun (26) is then moved forward the bag (7), it pierces it entering inside the same and, contemporarily, with the opening of the nozzle (29) and the inoculum and sterile air flow, the inoculum itself, inside the bag, becomes diffusely atomized on the whole surface of the substrate (12). Due to the inoculum injection and especially to the sterile air injection, between the gun barrel and the hole in the bag, a flow of coming out air arouses which prevents, during the inoculation step, the entrance of pollutants, inside the bag, the sterility being even more assured by the presence of the flame. While air is still coming out, the gun is drawn out and the hole is pluged by means of a large thickness pipe (8) of elastic, externally smooth, chemically sterilized, expanded plastic material with an internal vent wall covered with sterilizing product, besides an inwards check valve, if any, or a removable plug.

The device (8) for the air discharge from the bag (7) is located on the bag opposite side referred to the air entrance (9): it is placed in the bag lower side, so that the carbon dioxide, that is formed during the fermentation, being heavier than air, is helped to leave the bag.

The bag (7), inoculated and laid on the respective tray (23), is brought on one of the shelves (1) of the scaffolding (2) and here gently set down by turning upside down the tray (23) on a shelf (1). The bag (7) is immediately connected to the metallic nozzle (9) of the sterile injection air coming from the suitable filter (15), by flame heating and sterilizing the nozzle, then introduced into the bag (7) by wall melting. Air is immediately introduced into the bag, so contemporarily cooling the nozzle that is then fixed to the bag by a stick tape or by a small plastic band. Air can so flow above the medium (12) surface and go out through the discharge device (8).

A device (6), formed by two horizontal tubes, parallel to each other and to the plain, fixed on the metallic scaffolding (2), allows to keep partially pressed the bag (7), so maintaining reduced its cross section and then maintaining an optimum air velocity on the surface of the medium during fermentation and/or drying.

For a better understanding of the invention, reference is made to the attached Figures wherein:

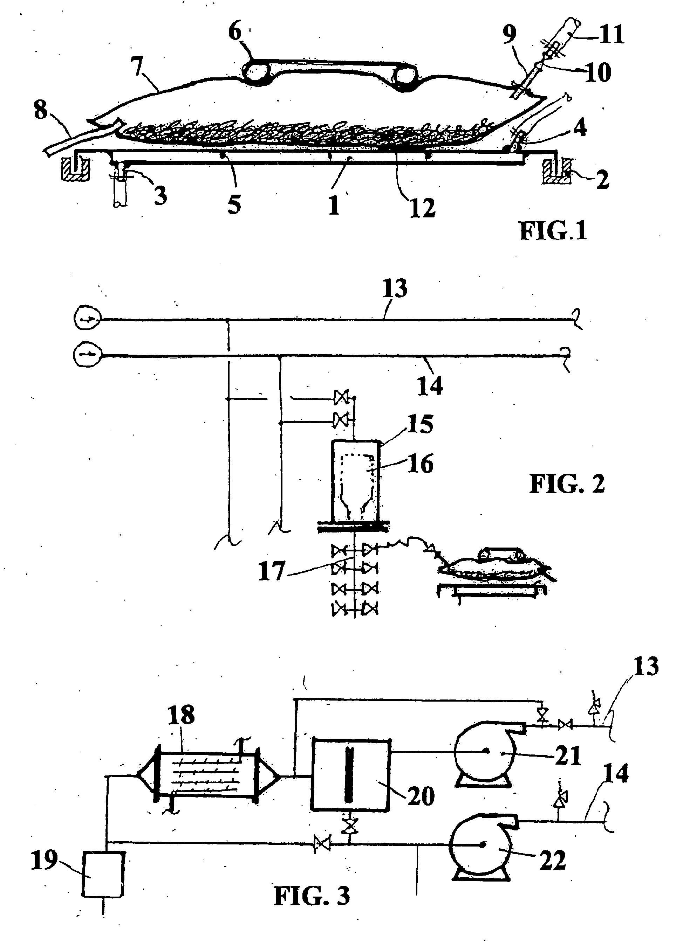

Figure 1 represents the fermentation and/or drying bag (7) on the thermostabilized shelf (1) of the scaffolding (2);
Figure 2 represents the humid (14) and dry air (13) manifolds, their connections with some of sterilizing filters (15), the manifold (17) on the filter itself and the connection between sterilizing filter and bag;
Figure 3 represents the equipment for the preparation of humid and dry air;
Firgure 4 represents the sterilizing autoclave (24) with the view of the room containing the rack (25) bearing trays with said trays (23);

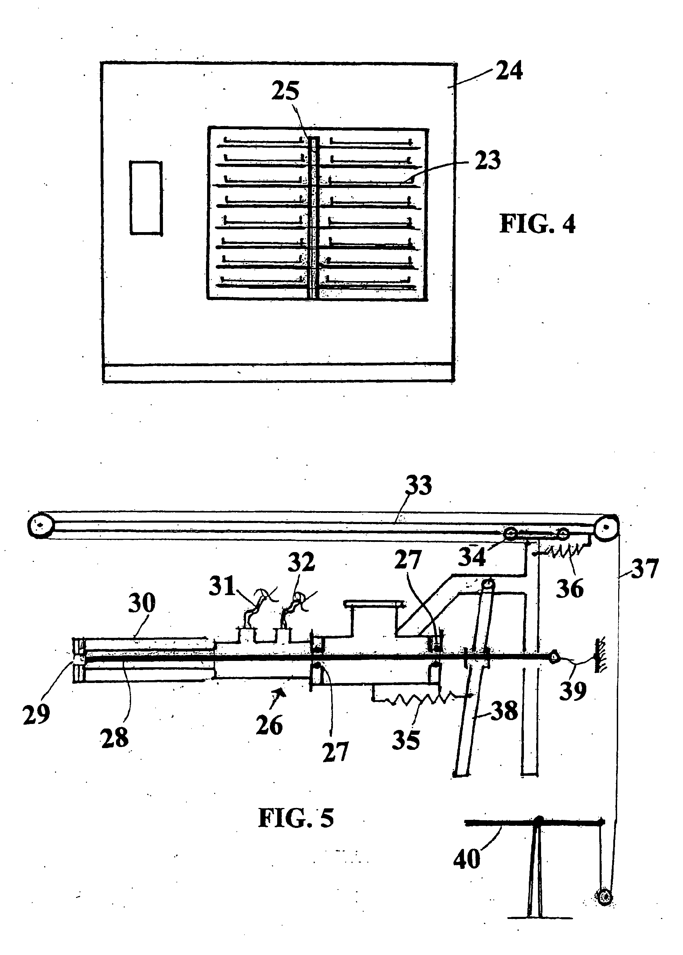

Figure 5 represents the injection gun (26) with the devices for feeding and for its handling. The plant embodiment of the present invention is built for the industrial production of eumycetes and/or bacilli spores, their drying and recover for the production of formulates for different uses such as agricultural, environmental uses or for the production of granules with biofixed enzymes, suitable to be introduced in reactors for enzymatic reactions.

## Claims

1. A solid state fermentation plant for the production of microorganisms and metabolites, **characterized by** the fact that is formed by:
- the closed scaffolding (2), of a transparent, termically insulating material, bearing the metallic shelves (1), on each one of them, the fermentation bags (7) are supported, the shelves (1) being provided with a suitable heating system selected between an interspace with buffles (5) and a coil;
- the autoclave (24) provided with the rack (25) carrying the trays (23);
- the automatic inoculum injection system into the hermetically closed bag (7) consisting of the stainless steel gun (26) having a double barrel (30) ending with the nozzle (29) and provided with the lock (28) and the relevant driving system consisting in the lever (38) with the return spring (35) and a seal chamber, containing a high boiling liquid, having two O-rings (27) around the lock (28); said gun (26) is connected, by the pipe (31), to the inoculum supply and, by the pipe (32), to the pressurized sterile air supply, and is able to heat pierce the bag (7) surface to inoculate the fermentation substrate (12); in the hole, which formed on the bag surface by injecting the inoculum, is fixed a suitably closeable piece of tube (8) to allow the fermentation air discharge;
- a humid (14) and dry (13) air production and supply system to the plant;
- a suitable number of air sterilizing filters (15) containing the replaceable sterilizing cartridges (16);
- a suitable number of air-tight closed bags (7) made of barrier, transparent, autoclave plastic material suitable to be heat pierced by the gun (26) and by the nozzle (9) for inoculum and sterile air injection, into the bag (7), containing the fermentation substrate (12);
- the pressure device (6) on the bag (7).

2. The solid state fermentation plant according to claim 1, **characterized by** the fact that the heat made holes by the barrel (30) of the gun (26) and by the nozzle (9) are each other diagonally opposed.

3. The solid state fermentation plant according to claim 1 or 2, **characterized by** the fact that the shelves (1) of the scaffolding (2) are made in stainless steel and at a distance of 40 cm one from the other.

4. The solid state fermentation plant according to any of claims 1 to 3, **characterized by** the fact that the trays (23) on the rack (25) are made in stainless steel perforated sheet.

5. The solid state fermentation plant according to any of claims 1 to 4, **characterized by** the fact that the ratio between the number of the shelves (1) and the number of the filters (15) is selected in the range 1-8.

6. The solid state fermentation plant according to any of claims 1 to 5, **characterized by** the fact that the pressure device (6) on the bag (7) is formed by two parallel tubes placed in a horizontal plane and transversally positioned with respect to the bag greatest axis.

7. The solid state fermentation plant according to any of claims 1 to 6, **characterized by** the fact that the gun (26) operates an electric signal for the supplying and metering the culture to be inoculated into the bag (7).

8. The use of the solid state fermentation plant as described in any of the previous claims for the production of microorganisms and metabolites.

## Patentansprüche

1. Feststofffermentationsanlage für die Produktion von Mikroorganismen und Stoffwechselprodukten, **dadurch gekennzeichnet, dass** sie gebildet ist, aus:
- dem geschlossenen Gerüst (2) aus einem transparentem, thermisch isolierendem Material, das die metallischen Einlegeböden (1) trägt, wobei auf jedem von diesen die Fermentationsbeutel (7) aufliegen, wobei die Einlegeböden (1) mit einem geeigneten Heizsystem versehen sind, das aus einem Zwischenraum mit Leitblechen und einer Wendel ausgewählt ist;
- dem Autoklav (24), der mit dem Regal (25) versehen ist, das die Tablette (23) trägt;
- dem automatische Inokulumimpfsystem in den hermetisch geschlossenen Beutel (7), das aus der rostfreien Stahlpistole (26) besteht, die einen doppelten Lauf (30) aufweist, der mit der Düse (29) endet und mit dem Verschluss (28) und dem relevanten Ansteuerungssystem versehen ist, das im Hebel (38) mit der Rückstellfeder (35) und einer Dichtkammer besteht, die eine hoch siedende Flüssigkeit enthält, die zwei O-Ringe (27) um den Verschluss (28) aufweist; wobei die Pistole (26) durch das Rohr (31) mit der Inokulumzufuhr und mit dem Rohr (32) mit dem Speicher unter Druck stehender steriler Luft verbunden und in der Lage ist, die Oberfläche des Beutels (7) heiß durchzustechen, um das Fermentationssubstrat (12) zu inokulieren; wobei ein geeignetes verschließbares Stück Schlauch (8) im Loch befestigt wird, das in der Beuteloberfläche durch Eindringen des Inokulums gebildet wird, um zu ermöglichen, dass die Fermentationsluft entweicht;
- einem Feucht-(14) und Trocken-(13)lufterzeugungs- und -zuführungssystem zu der Anlage;
- einer geeigneten Anzahl Luft sterilisierender Filter (15), welche die austauschbaren sterilisierenden Kartuschen (16) enthalten;
- einer geeigneten Anzahl luftdicht verschlossener Beutel (7), die aus transparentem Barriere-Autoklavenplastikmaterial hergestellt sind, das geeignet ist, von der Pistole (26) und von der Düse (9) für das Einbringen des Inokulums und der sterilen Luft in den Beutel (7) heiß durchstochen zu werden, der das Fermentationssubstrat (12) enthält;
- der Druckvorrichtung (6) an dem Beutel (7).

2. Feststofffermentationsanlage nach Anspruch 1, **dadurch gekennzeichnet**, das die durch den Lauf (30) der Pistole (26) und die Düse (9) hitzeerzeugten Löcher einander diagonal gegenüber liegen.

3. Feststofffermentationsanlage nach Anspruch 1 oder 2, **dadurch gekennzeichnet, dass** die Einlegeböden (1) des Gerüsts (2) aus rostfreiem Stahl hergestellt sind und sich einer Entfernung von 40 cm voneinander befinden.

4. Feststofffermentationsanlage nach einem der Ansprüche 1 bis 3, **dadurch gekennzeichnet, dass** die Tablette (23) auf dem Regal (25) aus perforierten rostfreien Stahlblechen hergestellt sind.

5. Feststofffermentationsanlage nach einem der Ansprüche 1 bis 4, **dadurch gekennzeichnet, dass** das Verhältnis zwischen der Anzahl an Einlegeböden (1) und der Anzahl an Filtern (15) in einem Bereich von 1 bis 8 gewählt ist.

6. Feststofffermentationsanlage nach einem der Ansprüche 1 bis 5, **dadurch gekennzeichnet, dass** die Druckvorrichtung (6) am Beutel (7) durch zwei parallele Schläuche gebildet wird, die in einer horizontalen Ebene und quer in Bezug auf die längste Achse des Beutels angeordnet sind.

7. Feststofffermentationsanlage nach einem der Ansprüche 1 bis 6, **dadurch gekennzeichnet, dass** die Pistole (26) ein elektrisches Signal zum Zuführen und Messen der in den Beutel (7) zu inokulierenden Kultur auslöst.

8. Verwendung der Feststofffermentationsanlage, wie in einem der vorhergegangenen Ansprüche beschrieben, zur Produktion von Mikroorganismen und Stoffwechselprodukten.

## Revendications

1. Installation de fermentation à l'état solid pour la production de microorganismes et métabolites, **caractérisée en ce qu'**elle est constituée de :
- le rayonnage fermé (2), de matériel transparent, thermiquement isolant, supportant les étagères métalliques (1), sur chacune desquelles, les sacs de fermentation (7) sont placés, les étagères (1) étantes pourvues d'un apte système de chauffage choisi entre en espace avec des diaphragmes (5) et un serpentin ;
- l'autoclave (24) pourvu du râtelier (25) supportant les plateaux (23) ;
- le système d'injection automatique de l'inocolum dans le sac (7) hermetiquement fermé consistant en le pistolet (26) en acier inoxydable, ayant a double canon (30) terminant avec le goulot (29) et pourvu de la culasse (28) et le correspondant système d'actionnement consistant en un levier (38) avec un ressort de retour (35) et en une chambre étanchée contenante un liquide haut bouillant, ayant deux anneaux toriques (27) autour de la culasse (28) ; ce pistolet (26) est relié, par le tube (31), à la fourniture de l'inoculum et, par le tube (32), à la fourniture de l'air stérile pressurisé et il est en mesure de perforer à chaud la surface du sac (7) pour inoculer le substrat de fermentation (12); sur le trou, formé sur la surface du sac injectant l'inocolum, est fixé un apte morceau de tube (8), qui peut être fermé, pour permettre la décharge de l'air de fermentation;
- une production d'air humide (14) et sec (13) et le système de fourniture à l'installation ;
- un apte nombre de filtres stérirsantes (15) contenants les cartouches remplaçables stérisisantes (16) ;
- un apte nombre da sacs (7) fermés étanches à l'air faits de matériel plastique, barrière, transparents, pour autoclave, apte pour être perforé à chaud par le pistolet (26) et par le goulot (9) pour l'injection de l'inoculum et de l'air stérile dans le sac (7), contenant le substrat de fermentation (12);
- le dispositif de pression (6) sur le sac (7) ;

2. Installation de fermentation à l'état solide selon la revendication 1, **caractérisée en ce que** les trous faits par chaleur par le canon (30) du pistolet (26) et par le goulot (9) sont mutuellement diagonalement opposés.

3. Installation de fermentation à l'état solide selon la revendication 1 or 2, **caractérisée en ce que** les étagères (1) du rayonnage (2) sont faits en acier inoxydable et à une distance de 40 cm l'une de l'autre.

4. Installation de fermentation à l'état solide selon une quelconque des revendications 1 à 3, **caracterisée en ce que** les plateaux (23) sur le râtelier (25) sont faits en tôle perforée d'acier inoxydable.

5. Installation de fermentation à l'etat solide selon une quelconque des revendications 1 à 4, **caractérisée en ce que** le rapport entre le nombre des étagères (1) et le nombre des filtres (15) est choisi dans l'intervalle 1-8.

6. Installation de fermentation à l'état solide selon une quelconque des revendications 1 à 5, **caractérisée en ce que** le dispositif de pression (6) sur le sac (7) est formé de deux tubes parallèles disposés dans un plan horizontal et positionnés de travers par rapport à l'axe majeur du sac.

7. Installation de fermentation à l'etat solide selon une quelconque des revendications 1 à 6, **caractérisée en ce que** le pistolet (26) fournit un signal électrique pour la fourniture et la mesure de la culture d'inoculer dans le sac (7).

8. Usage d'une installation de fermentation à l'état solide comme décrite dans u0ne quelconque des revendications précédentes pour la production de microorganismes et metabolites.
